**Europäisches Patentamt**

(19) **European Patent Office**

**Office européen des brevets**

(11) Numéro de publication: **0 053 055**
**B1**

(12) ## FASCICULE DE BREVET EUROPEEN

(45) Date de publication du fascicule du brevet:
**18.07.84**

(21) Numéro de dépôt: **81401687.9**

(22) Date de dépôt: **23.10.81**

(51) Int. Cl.³: **C 11 D 17/00,** C 11 D 3/50,
A 61 L 9/01

(54) **Bloc solide pour la désodorisation et le nettoyage de cuvettes de toilettes.**

(30) Priorité: **20.11.80 FR 8024668**

(43) Date de publication de la demande:
**02.06.82 Bulletin 82/22**

(45) Mention de la délivrance du brevet:
**18.07.84 Bulletin 84/29**

(84) Etats contractants désignés:
**AT BE CH DE GB IT LI LU NL SE**

(56) Documents cités:
**EP - A - 0 014 979**
**FR - A - 2 309 619**
**GB - A - 774 407**
**US - A - 4 149 986**

(73) Titulaire: **L'OREAL Société anonyme dite:, 14, Rue
Royale, F-75008 Paris (FR)**

(72) Inventeur: **Cadoret, Philippe, 2, allée A. Honegger,
F-95250 Beauchamp (FR)**
Inventeur: **Verite, Claude, 4, rue Jacques-Coeur,
F-75004 Paris (FR)**
Inventeur: **Chesbeuf, Bernard, 1, Chemin des Fauvettes,
Parc Corot F-93470 Coubron (FR)**

(74) Mandataire: **Peuscet, Jacques, 3, Square de Maubeuge,
F-75009 Paris (FR)**

## Description

La présente invention concerne une composition pour la désodorisation et le nettoyage de cuvettes de toilettes, se présentant sous forme solide à la température ambiante, ladite composition contenant une quantité efficace de détergent(s) et, en outre, du parfum et d'autres additifs usuels.

Une composition de ce genre se présente généralement sous la forme d'un bloc et, en particulier, d'un bâton placé dans un support que l'on accroche à l'intérieur de la cuvette de toilettes, dans un endroit où il peut être arrosé par l'eau de chasse. A chaque opération de chasse, l'eau entraîne une petite quantité de produit désodorisant et nettoyant contenu dans le bâton, jusqu'à usure complète dudit bâton.

Ces blocs de produit peuvent être pour l'essentiel fabriqués selon deux techniques: d'une part, l'extrusion après mélange à sec et/ou broyage des constituants selon le procédé d'obtention des savons et, d'autre part, le coulage à chaud à l'intérieur d'un moule après mélange en phase liquide de l'ensemble des constituants. Le coulage à chaud est suivi d'une cristallisation dont la vitesse peut être accélérée par un refroidissement intensif.

Cette dernière technique, si elle est couramment utilisée, nécessite cependant que la formulation soit sensiblement exempte d'eau pour éviter des problèmes de cristallisation et, par suite, pour favoriser le démoulage du bloc de produit solidifié. On a en effet constaté que des formulations qui renfermaient de l'eau donnaient naissance à des blocs coulés d'aspect peu esthétique en raison de fendillements se produisant à la solidification et ne présentant pas les caractéristiques requises du fait notamment de leur usure trop rapide en cours d'usage.

C'est ainsi que, jusqu'à présent, les formulations courantes destinées à être coulées à chaud ne contenaient que des constituants sensiblement exempts d'eau, et en particulier des tensio-actifs anioniques tels que des alkylaryl sulfonates en poudre ou en écailles, des alkyl silfates en poudre, des paraffine sulfonates en écailles, des éther sulfates en pâte, c'est-à-dire des produits pratiquement purs ne contenant qu'une faible quantité de sels minéraux et éventuellement d'eau résultant du caractère hygroscopique de ces matières. Elles pouvaient aussi centenir des tensio-actifs non ioniques tels que des éthoxylats d'alcool gras, des stabilisants de mousse comme le monoéthanolamide de coprah. Parmi les autres additifs sensiblement exempts d'eau pouvant être incorporés dans des compositions coulables à chaud, on peut encore citer les polyéthylène glycols et les copolymères d'oxyde d'éthylène et d'oxyde de propylène.

La présente invention a pour but de décrire une nouvelle composition coulée à chaud à l'intérieur d'un moule qui, bien que préparée à partir de composants aqueux, constitue des blocs désodorisants et nettoyants ayant les mêmes caractéristiques, en ce qui concerne l'aspect du produit et les qualités d'utilisation, que les blocs semblables exempts d'eau de l'état de la technique.

La présente invention a donc pour objet un bloc solide à la température ambiante et destiné à la désodorisation et au nettoyage de cuvettes de toilettes, ledit bloc étant obtenu par coulée dans un moule à une température comprise entre 65 et 70°C et contenant une quantité efficace de détergent, du parfum, de l'eau, un sel minéral et des additifs usuels, caractérisé par le fait qu'il contient de 4 à 16,66% d'eau, et, comme sel minéral, de 2 à 5% de clorure de calcium, les pourcentages ci-dessus étant indiqués en poids par rapport au poids total du bloc.

La société déposante a en effet constaté que de façon surprenante, l'addition d'une quantité suffisante de clorure de calcium, anhydre ou partiellement hydraté, susceptible de s'hydrater rapidement à la température du mélange, autorisait l'emploi de constituants non purifiés pouvant contenir une forte proportion d'eau et par suite, de faible prix de revient, sans que cela nuise aux qualités esthétiques et d'utilisation des blocs obtenus.

Le pourcentage de chlorure de calcium anhydre ou partiellement hydraté à incorporer dans le bloc est fonction de la teneur en eau du bloc. Si la quantité de chlorure de calcium est trop faible, le bloc obtenu ne présente pas les caractéristiques requises; par contre, une quantité trop élevée de chlorure de calcium modifie la viscosité en phase liquide lors du mélange des constituants, de sorte que la température de coulée du bloc doit être trop élevée, ce qui a pour effet de rendre le bloc coulé pratiquement inusable et, par suite, sans aucune qualité à l'utilisation. La société déposante a donc déterminé les quantités relatives d'eau et de chlorure de calcium qui ont été ci-dessus indiquées et qui permettent d'atteindre des résultats satisfaisants.

La quantité d'eau contenue dans la composition selon l'invention peut être apportée par un ou plusieurs constituants aqueux. Selon une caractéristique avantageuse, la composition selon l'invention contient comme détergent anionique, un ether sulfate incorporé à l'état aqueux dans ladite composition. L'éther sulfate utilisé est d'une façon générale un sel d'un alcool en $C_{12}-C_{16}$ polyoxyéthyléné et sulfaté. L'ion générateur du sel est avantageusement un métal alcalin tel que le sodium ou le potassium et est, de préférence, du sodium. On préfère le sel de sodium de l'alcool en $C_{12}-C_{16}$ oxyéthyléné (à 3 môles d'oxyde d'éthylène pour une môle d'alcool) et sulfaté.

L'éther sulfate aqueux qui est incorporé à la composition selon l'invention renferme de 20 à 40% et plus avantageusement environ 30% en poids d'eau.

Pour l'obtention d'un bon pouvoir moussant et d'une bonne tenue du bloc à l'usure, on a constaté que le pourcentage d'éther sulfate devait être compris entre 25% et 35% en poids par rapport au poids total de l'ensemble des constituants entrant dans la composition.

La composition selon l'invention contient de façon classique, au moins un colorant destiné à colorer l'eau de chasse, mais ce colorant est avantageusement ajouté sous forme de solution aqueuse par exemple de solution à 50%. Dans ce cas également, la quantité d'eau présente dans le colorant aqueux est absorbée par le chlorure de calcium et ne soulève par conséquent aucune difficulté.

Une autre caractéristique avantageuse de la composition selon l'invention est qu'elle peut renfermer une quantité élevée de parfum liquide pouvant aller jusqu'à 10% en poids par rapport au poids total de la composition.

De préférence, la composition selon l'invention contient d'environ 20 à environ 50% de paradichlorobenzène; d'environ 16 à environ 37% de polyéthylène glycol; d'environ 0 à environ 8% de monoéthanolamide de coprah; d'environ 25 à environ 35% d'éther sulfate; d'environ 2 à environ 5% de chlorure de calcium; et d'environ 3 à environ 12% de parfum et de colorant en solution, ces pourcentages étant exprimés en poids par rapport au poids total de la composition.

La société déposante a en effet découvert que, lorsque le mélange des constituants et le coulage du liquide obtenu étaient effectués dans la gamme de températures 65° −70°C, le bloc de produit obtenu présentait une bonne cohésion et pouvait s'user progressivement sans se désagréger. On préfère que la gamme de température précitée aille de 67°C à 70°C.

Avantageusement, on effectue le mélange à une température d'environ 65°C à l'intérieur d'une cuve de mélange, l'incorporation du chlorure de calcium anhydre, du colorant en solution et du parfum étant réalisée en dernier lieu. L'introduction du chlorure de calcium anhydre ou partiellement hydraté provoque une élévation de température, de sorte que le mélange liquide est coulé à environ 70°C dans des moules ayant la forme du produit solide que l'on désire obtenir. De façon classique, les moules passent ensuite dans un tunnel de refroidissement, ce qui a pour effet d'accélérer la vitesse de cristallisation du produit.

Pour mieux faire comprendre l'objet de l'invention, on va en donner ci-après, à titre purement illustratif et non limitatif, plusieurs exemples de formulations désodorisantes et nettoyantes.

Pour fabriquer par coulage à chaud des blocs de produit désodorisant et nettoyant, on effectue dans les exemples suivants, un mélange homogène des divers constituants dans les rapports pondéraux et selon l'ordre d'introduction ci-après indiqués. Les constituants de la formulation sont mélangés, en phase liquide, à une température d'environ 65°C. L'introduction de chlorure de calcium provoque une élévation de température et le produit liquide est finalement coulé à environ 70°C dans des moules qui passent ensuite dans un tunnel de refroidissement. L'éther silfate utilisé est le sel de sodium de l'alcool en $C_{12}-C_{16}$ oxyéthyléné (à 3 môles d'oxyde d'éthylène pour une môle d'alcool) et sulfaté.

### Exemple 1

| | |
|---|---|
| — Paradichlorobenzène | 20% |
| — Polyéthylène glycol (de poids moléculaire 8500) | 35% |
| — Monoéthanolamide de coprah | 8% |
| — Ether sulfate aqueux à 70% de matières actives | 30% |
| — Chlorure de calcium en poudre | 3,5% |
| — Colorant en solution dans de propylène glycol | 0,5% |
| — Parfum | 3% |
| | 100% |

### Exemple 2

| | |
|---|---|
| — Paradichlorobenzène | 20% |
| — Polyéthylène glycol (poids moléculaire 8500) | 26% |
| — Monoéthanolamide de coprah | 8% |
| — Ether sulfate aqueux à 70% de matières actives | 30% |
| — Chlorure de calcium en poudre | 4% |
| — Colorant en solution dans l'eau | 4% |
| — Parfum | 8% |
| | 100% |

0 053 055

Exemple 3

| — | Paradichlorobenzène | 50% |
|---|---|---|
| — | Polyéthylène glycol (poids moléculaire 8500) | 16,9% |
| — | Ether sulfate aqueux à 70% matières actives | 27% |
| — | Chlorure de calcium | 3,1% |
| — | Parfum, colorant | 3% |
| | | 100% |

Exemple 4

| — | Paradichlorobenzène | 30% |
|---|---|---|
| — | Polyéthylène glycol (poids moléculaire 8500) | 36,9% |
| — | Ether sulfate aqueux à 70% de matières actives | 27% |
| — | Chlorure de calcium | 3,1% |
| — | Parfum, colorant | 3% |
| | | 100% |

Il est bien entendu que les modes de réalisation ci-dessus décrits ne sont aucunement limitatifs et pourront donner lieu à toute modification désirable sans dortir pour cela du cadre de l'invention défini par les revendications.

**Revendications**

1. Bloc solide à la température ambiante et destiné à la désodorisation et au nettoyage de cuvettes de toilettes, ledit bloc étant obtenu par coulée dans un moule à une température comprise entre 65 et 70°C et contenant une quantité efficace de détergent, du parfum, de l'eau, un sel minéral et des additifs usuels, caractérisé par le fait qu'il contient de 4 à 16,66% d'eau, et, comme sel minéral, de 2 à 5% de chlorure de calcium, les pourcentages ci-dessus étant indiqués en poids par rapport au poids total du bloc.

2. Bloc selon la revendication 1, caractérisé par le fait qu'il contient, comme détergent, un détergent anionique.

3. Bloc selon la revendication 2, caractérisé par le fait que le détergent anionique est un éther-sulfate.

4. Bloc selon la revendication 3, caractérisé par le fait que l'éther-sulfate qu'il contient est un sel d'un alcool en $C_{12}-C_{16}$ polyoxyéthyléné et sulfaté.

5. Bloc selon l'une des revendications 3 ou 4, caractérisé par le fait qu'il contient de 25% à 35% d'éther-sulfate, ces pourcentages étant exprimés en poids par rapport au poids total du bloc.

6. Bloc selon l'une des revendications 1 à 5, caractérisé par le fait qu'il contient de 1 à 10% en poids de parfum par rapport au poids total du bloc.

7. Bloc selon l'une des revendications 1 à 6, caractérisé par le fait qu'il contient de 20 à 50% de paradichlorobenzène, de 16 à 37% de polyéthylène glycol, de 0 à 8% de monoéthanolamide de coprah et de 3 à 12% de colorant, tous les pourcentages ci-dessus étant indiqués en poids par rapport au poids total du bloc.

8. Bloc selon l'une des revendications 1 à 7, caractérisé par le fait qu'il est coulé à une température comprise entre 67 et 70°C.

**Patentansprüche**

1. Bei Raumtemperatur fester und zur Desodorierung und Reinigung von Toilettenbecken bestimmter Block, wobei dieser Block durch Gießen in eine Form bei einer Temperatur zwischen 65 und 70°C erhalten wird und eine ausreichende Menge Detergens, Parfum, Wasser, ein Mineralsalz und übliche Additive enthält, dadurch gekennzeichnet, daß er 4 bis 16,66% Wasser und als Mineralsalz 2 bis 5% Calciumchlorid enthält, angegeben als Gewichtsprozent, bezogen auf das Gesamtgewicht des Blocks.

2. Block nach Anspruch 1, dadurch gekennzeichnet, daß er als Detergens ein anionisches Detergens enthält.

3. Block nach Anspruch 2, dadurch gekennzeichnet, daß er als anionisches Detergens ein Äthersulfat enthält.

4. Block nach Anspruch 3, dadurch gekennzeichnet, daß es sich bei dem im Block enthaltenen Äthersulfat um ein Salz eines polyoxyethylenierten und sulfatierten $C_{12}-C_{16}$-Alkohols handelt.

5. Block nach einem der Ansprüche 3 oder 4, dadurch gekennzeichnet, daß er 25 bis 35% Äthersulfat enthält, ausgedrückt in Gewichtsprozenten, bezogen auf das Gesamtgewicht des Blocks.

6. Block nach einem der Ansprüche 1 bis 5, dadurch gekennzeichnet, daß er 1 bis 10 Gew.-% Parfum enthält, bezogen auf das Gesamtgewicht des Blocks.

7. Block nach einem der Ansprüche 1 bis 6, dadurch gekennzeichnet, daß er 20 bis 50% p-Dichlorbenzol, 16 bis 37% Polyethylenglykol, 0 bis 8% Kopramonoethanolamid und 3 bis 12% Farbstoff enthält, wobei alle Prozentangaben ausgedrückt sind als Gewichtsprozente, bezogen auf das Gesamtgewicht des Mittels.

8. Block nach einem der Ansprüche 1 bis 7, dadurch gekennzeichnet, daß er bei einer Temperatur zwischen 67 und 70° C gegossen wird.

## Claims

1. A block solid at the ambient temperature and intended for the deodorisation and cleaning of toilet pans, the said block being obtained by casting in a mould at a temperature comprised between 65 and 70°C and containing an adequate quantity of detergent, of perfume, of water a mineral salt and the usual additives, characterised in that it contains 4 to 16.6% of water, and, as mineral salt, 2 to 5% calcium chloride, the above mentioned percentages being indicated in terms of weight in relation to the total weight of the block.

2. A block according to claim 1, characterised in that it contains an anionic detergent as detergent.

3. A block according to claim 2, characterised in that the anionic detergent is an ether sulphate.

4. A block according to claim 3, characterised in that the ether sulphate which it contains is a salt of a sulphated $C_{12}-C_{16}$ polyoxyethylene alcohol.

5. A block according to one of claims 3 or 4, characterised in that it contains from 25% to 35% of ether sulphate, these percentages being expressed in terms of weight in relation to the total weight of the block.

6. A block according to one of claims 1 to 5, characterised in that it contains 1 to 10% by weight of perfume in relation to the total weight of the block.

7. A block according to one of claims 1 to 6, characterised in that it contains 20 to 50% of paradichlorobenzene, 16—37% of polyethylene glykol, 0 to 8% of monoethanolamide of copra and 3 to 12% of colouring agent, all the above percentages being indicated by weight in relation to the total weight of the block.

8. A block according to one of claims 1 to 7, characterised in that it is cast at a temperature comprised between 67 and 70°C.